# FASCICULE DE BREVET EUROPEEN

(11) **EP 4 003 440 B1**
(45) Date de publication et mention de la délivrance du brevet: **05.03.2025**
(21) Numéro de dépôt: 20742740.2
(22) Date de dépôt: 22.07.2020
(51) Int. Cl.: A61L 27/36, A61L 27/38, A23L 5/44, A61K 8/73, A61K 31/01, A61K 31/736, A61K 36/9066

(54) **PROCEDE DE PREPARATION D'UN MATERIAU D'ALLOGREFFE OU DE XENOGREFFE A PARTIR D'UNE CAPSULE CRISTALLINIENNE**
VERFAHREN ZUR HERSTELLUNG EINES ALLOTRANSPLANTATS ODER XENOTRANSPLANTATS AUS EINER KRISTALLINEN LINSENKAPSEL
METHOD FOR PREPARING AN ALLOGRAFT OR XENOGRAFT MATERIAL FROM A CRYSTALLINE LENS CAPSULE

(30) Priorité: 22.07.2019 FR 1908275
(43) Date de publication de la demande: 01.06.2022
(73) Titulaire: Université Jean Monnet Saint-Étienne, 42100 Saint-Étienne (FR); Centre Hospitalier Universitaire, 42100 Saint Etienne (FR)
(72) Inventeur: GAIN, Philippe, 69009 LYON (FR); THURET, Gilles, 42330 ST BONNET LES OULES (FR); HE, Zhiguo, 42100 SAINT ETIENNE (FR)
(74) Mandataire: Be IP
(86) Numéro de dépôt international: PCT/EP2020/070692
(87) Numéro de publication internationale: WO 2021/013893

(56) Documents cités:
- WO-A1-2018/102329
- US-A1- 2002 183 844
- T. EBERLEIN ET AL: "Clinical Use of Polihexanide on Acute and Chronic Wounds for Antisepsis and Decontamination", SKIN PHARMACOLOGY AND PHYSIOLOGY, vol. 23, no. 1, 1 January 2010 (2010-01-01), CH, pages 45 - 51, XP055735221, ISSN: 1660-5527, DOI: 10.1159/000318267

## Description

### DOMAINE DE L'INVENTION

La présente invention concerne le domaine technique général de la production d'un greffon - humain ou animal - implantable pour des applications ophtalmologiques.

Plus précisément, l'invention concerne un procédé de préparation d'un matériau d'allogreffe ou de xénogreffe formant une membrane, lyophilisée et stérile issue d'une capsule cristallinienne.

Ce matériau d'allogreffe ou de xénogreffe est adapté pour servir de greffon en chirurgie oculaire, notamment dans le cadre d'une chirurgie cornéenne et/ou rétinienne.

### ARRIERE PLAN DE L'INVENTION

La capsule cristallinienne est une membrane transparente et résistante enveloppant le cristallin.

A l'heure actuelle, les capsules cristalliniennes sont considérées comme des rebuts opératoires. Notamment lors d'une chirurgie de la cataracte, un disque de capsule cristallinienne antérieure (ci-après dénommée « *capsule antérieure* ») est découpé, par exemple à l'aide d'instruments de découpe mécanisés (tel qu'un L.A.S.E.R. femtoseconde, ou tout autre instrument de découpe connu de l'homme du métier). Ce disque de capsule antérieure - d'un diamètre sensiblement égal à 6 millimètres - constitue un déchet opératoire qui est détruit de manière systématique.

Or la capsule cristallinienne, qui est composée principalement de collagène IV, constitue un excellent support pour cultiver différents types de cellules utiles afin de produire des matériaux de thérapie cellulaire et tissulaire tels que des greffons endothéliaux cornéens ou épithéliaux cornéens ou des greffons rétiniens (épithélium pigmenté, photorécepteurs, etc.).

Les capsules antérieures issues d'une opération de cataracte (850 000 opérations par an en France, dont au moins 10% sont réalisées en utilisant un instrument de découpe mécanisé pour découper la capsule antérieure) pourraient être utilisées pour réaliser des matériaux d'allogreffe ou de xénogreffe.

Une autre source d'approvisionnement en capsule cristallinienne concerne les dons de tissus et d'organes, en particulier de cornées ou des globes oculaires entiers, pour lesquels des disques de capsules cristalliniennes antérieure et postérieure pourraient être découpés sur des cristallins préalablement prélevés, cette solution d'approvisionnement permettant d'une part la découpe de disques de diamètres plus important (compris entre 7 et 11 millimètres), et d'autre part de récupérer à la fois la capsule cristallinienne antérieure et la capsule cristallinienne postérieure.

Le document US 2002/183844 concerne un tissu micro-fabriqué formant substrat pour une transplantation d'épithélium pigmentaire, et son procédé de fabrication. Le procédé comprend les étapes consistant à modifier un tissu membraneux, et à faire croître des cellules sur le tissu membraneux modifié. Le procédé décrit dans US 2002/183844 permet de modifier le tissu membraneux afin de favoriser l'adhérence cellulaire sur celui-ci.

Aucun procédé industriel de préparation d'un matériau d'allogreffe ou de xénogreffe à partir d'une capsule cristallinienne découpée, par exemple à l'aide d'instruments de découpe mécanisés (tel qu'un L.A.S.E.R. ultrabref), ou tout autre instrument de découpe électrique ou mécanique ou manuelle n'a jusqu'à présent été proposé.

Un but de la présente invention est de remédier à cet inconvénient.

### BREVE DESCRIPTION DE L'INVENTION

A cet effet, l'invention propose un procédé de préparation d'un matériau d'allogreffe ou de xénogreffe à partir d'une capsule cristallinienne, comprenant les étapes suivantes :
- Dé-cellularisation (200) d'une capsule cristallinienne (71) pour obtenir une capsule cristallinienne dé-cellularisée,
- Dépôt (300) de la capsule dé-cellularisée sur une membrane support (31) microporeuse pour obtenir un empilement composé de la capsule cristallinienne dé-cellularisée et de la membrane support,
- Marquage de la capsule cristallinienne dé-cellularisée pour former des moyens repère d'orientation, ladite étape de marquage consistant en l'application d'une encre biocompatible à travers la membrane support (31),
- Lyophilisation (400) de l'empilement pour obtenir un empilement lyophilisé,
- Stérilisation (500) de l'empilement lyophilisée pour obtenir un empilement stérilisé,
- Emballage (600) de l'empilement stérilisé pour obtenir des matériaux d'allogreffe ou de xénogreffe.

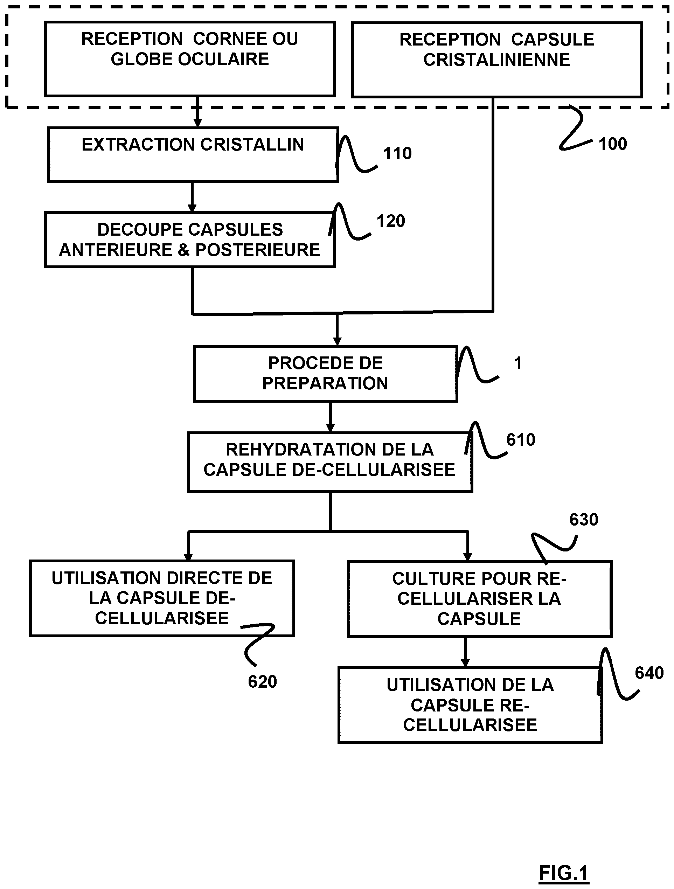

Ainsi et comme illustré aux étapes 610, 620 de la figure 1, le procédé selon l'invention permet d'avoir à disposition dans un conteneur stérile une capsule dé-cellularisée, facile à manipuler pour une chirurgie oculaire de type :
- fermeture d'un défect endothélial cornéen : il s'agit d'un mode de traitement d'une pathologie cornéenne fréquente appelée dystrophie endothéliale de Fuchs, caractérisée par la présence de structures anormales de la membrane de Descemet située à la face postérieure de la cornée centrale. Le chirurgien enlève alors mécaniquement cette couche anomale par un geste appelé Descemétorhexis. Ce Descemétorhexis laisse un défect dans l'endothélium qui est lentement comblé par les cellules endothéliales qui migrent depuis la périphérie. Afin d'accélérer la migration de ces cellules, il est possible de couvrir le défect par une membrane fine, transparente et favorable à l'adhérence et la migration des cellules. La capsule cristallinienne dé-cellularisée possède toutes ces qualités.
- **fermeture** d'un trou maculaire (TM) rétinien : cette pathologie rétinienne fréquente peut bénéficier, après réalisation d'une vitrectomie, de la pose d'une capsule cristallinienne dé-cellularisée afin de faciliter l'obturation du trou.

Dans ces deux applications la capsule dé-cellularisée est utilisée telle que, sans étape supplémentaire de re-cellularisation.

En variante et comme illustré aux étapes 630 et 640 de la figure 1, la capsule dé-cellularisée stérilisée peut être re-cellularisée avec des cellules endothéliales ou épithéliales cornéennes, ou des cellules de l'épithélium rétinien pigmenté ou d'autre cellules rétinienne telles photorécepteurs, cellules ganglionnaires etc. pour obtenir un greffon re-cellularisé, contenu dans un milieu de culture, prêt à la greffe.

Des aspects préférés mais non limitatifs du procédé selon l'invention sont les suivants :
- le procédé peut comprendre en outre une étape de découpe de la capsule cristallinienne pour former des moyens de préhension ;
- le procédé peut comprendre, préalablement à l'étape de dé-cellularisation :
   o une étape de réception d'un matériel biologique primaire choisi parmi : un globe oculaire ou une cornée issu d'un don d'organe, ou une capsule cristallinienne issue d'une opération de la cataracte et constituant un rebut opératoire,
   o si le matériau biologique est un globe ou une cornée :
      ▪ une étape d'extraction du cristallin,
      ▪ une étape de découpe des capsules cristalliniennes antérieure et postérieure ;
- l'étape de découpe des capsules cristalliniennes antérieure et postérieure comprend les sous-étapes consistant à :
   o insérer le cristallin entier dans un dispositif de maintien incluant un logement transparent au rayonnement L.A.S.E.R., et des moyens d'aspiration pour plaquer le cristallin dans le logement,
   o découper en utilisant un rayonnement L.A.S.E.R., la face antérieure de la capsule cristallinienne pour obtenir un disque découpé antérieur,
   o découper en utilisant le rayonnement L.A.S.E.R., la face postérieure de la capsule cristallinienne pour obtenir un disque découpé postérieur,
   o disséquer les disques antérieur et postérieur découpés pour obtenir une capsule cristallinienne antérieure et une capsule cristallinienne postérieure ;
- le procédé peut comprendre en outre une étape de marquage de la capsule cristallinienne pour former des moyens repère d'orientation, ladite étape de marquage consistant en l'application d'une encre biocompatible à travers la membrane support ;
- l'étape de dé-cellularisation peut comprendre les sous-étapes suivantes consistant à :
   o immerger la capsule cristallinienne dans un liquide de dé-cellularisation, et
   ∘ optionnellement soumettre la capsule immergée à des vibrations mécaniques et/ou gratter mécaniquement la surface de la capsule cristallinienne,
   ∘ rincer la capsule cristallinienne avec un liquide de rinçage (tel qu'une solution sérum physiologique) ;
- l'étape de dépôt peut comprendre les sous-étapes consistant à :
   o positionner la capsule cristallinienne à plat sur la membrane support,
   o effectuer une dessication de la capsule cristallinienne pour favoriser l'adhérence de la capsule cristallinienne sur la membrane support ;
- le procédé peut également comprendre, postérieurement à l'étape d'emballage, une étape de culture cellulaire ou tissulaire, ladite étape de culture incluant les sous-étapes consistant à :
   o enduire la capsule cristallinienne avec une substance favorisant l'adhérence des cellules/tissus destinés à être cultivés,
   o faire croître des cellules/tissus sur la capsule cristallinienne ;
- le procédé peut également comprendre, postérieurement à l'étape d'emballage, une étape de réhydratation du matériau d'allogreffe ou de xénogreffe, pour permettre l'utilisation de la capsule cristallinienne soit directement en tant qu'agent cicatrisant, soit postérieurement à la mise en œuvre d'une étape de culture cellulaire ou tissulaire.

L'invention concerne également un kit chirurgical pour le traitement d'une pathologie oculaire, ***remarquable en ce que*** le kit comprend un matériau d'allogreffe ou de xénogreffe obtenu en mettant en œuvre le procédé décrit ci-dessus.

### BREVE DESCRIPTION DES DESSINS

D'autres avantages et caractéristiques du procédé selon l'invention ressortiront mieux de la description qui va suivre de plusieurs variantes d'exécution, données à titre d'exemples non limitatifs, à partir des dessins annexés sur lesquels :
- La figure 1 est une représentation schématique :
   o des différentes sources d'approvisionnement possibles en capsules cristalliniennes, ainsi que
   o des différentes utilisations possibles des capsules cristalliniennes préparées à partir du procédé de préparation selon l'invention,
- La figure 2 est une représentation schématique des étapes du procédé de préparation selon l'invention,
- La figure 3 est une représentation schématique en coupe d'un dispositif de maintien d'un cristallin,
- La figure 4 est une représentation schématique en vue de dessus du dispositif de maintien du cristallin,
- La figure 5 est une représentation schématique de différentes formes de capsules cristalliniennes découpées,
- La figure 6 est une représentation schématique de différents aspects d'un dispositif de culture cellulaire,
- La figure 7 est une représentation schématique d'un logement du dispositif de culture cellulaire,
- La figure 8 est une représentation schématique d'une étape de marquage d'une capsule cristallinienne,
- La figure 9 est une représentation schématique de sous-étapes de culture d'une capsule cristallinienne préparée à partir du procédé de préparation selon l'invention.

### DESCRIPTION DETAILLEE DE L'INVENTION

On va maintenant décrire différents exemples du procédé de préparation d'un matériau d'allogreffe ou de xénogreffe en référence aux figures. Dans ces différentes figures, les éléments équivalents sont désignés par la même référence numérique.

### 1. Généralités

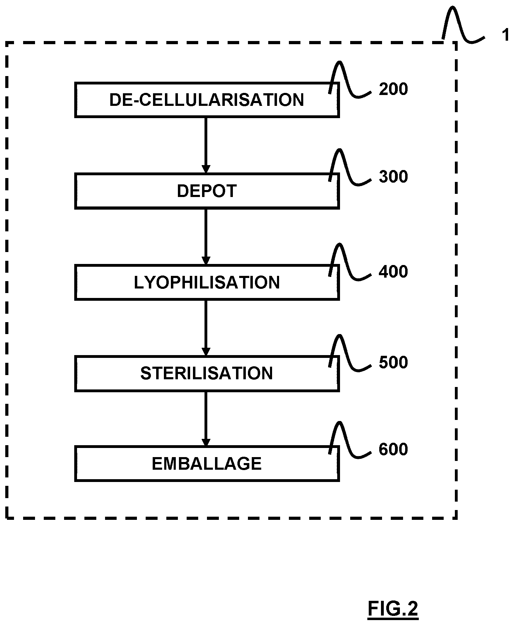

En référence à la figure 2, le procédé 1 comprend les étapes suivantes :
- Dé-cellularisation 200 de chaque capsule cristallinienne pour obtenir des capsules cristalliniennes dé-cellularisées,
- Dépôt 300 de chaque capsule à plat sur un support synthétique par exemple de type membrane microporeuse d'insert de culture cellulaire,
- Lyophilisation 400 de chaque capsule cristallinienne pour obtenir des capsules cristalliniennes lyophilisées (la lyophilisation se faisant de préférence directement avec le support synthétique, mais pouvant aussi être réalisée avec la capsule seule),
- Stérilisation 500 de chaque capsule cristallinienne lyophilisée pour obtenir des capsules cristalliniennes stérilisées (donc avec ou sans le support synthétique),
- Emballage et stockage 600 de chaque capsule cristallinienne stérilisée pour obtenir des matériaux d'allogreffe ou de xénogreffe.

Comme il sera décrit plus en détails au point 3 ci-dessous, le matériau d'allogreffe ou de xénogreffe ainsi obtenu (i.e. capsule cristallinienne dé-cellularisée, lyophilisée, stérilisée et emballée) peut être utilisé pour la réalisation d'une greffe. Plus précisément, la capsule cristallinienne dé-cellularisée peut être utilisée :
- Soit directement après la mise en œuvre d'une étape de réhydratation 610,
- Soit postérieurement à la mise en œuvre d'une étape de culture 630.

### 2. Description détaillée des étapes du procédé de préparation

### 2.1. Réception de matériau biologique

Les capsules cristalliniennes préparées à partir du procédé de préparation selon l'invention peuvent être issues de différentes sources d'approvisionnement. En particulier, ces capsules cristalliniennes peuvent être issues :
- de dons de cornées ou de globes oculaires, ou
- de capsules cristalliniennes découpées sur des patients opérés de la cataracte.

En fonction de la source d'approvisionnement, le procédé de préparation peut comprendre des étapes préalables à l'étape de dé-cellularisation.

### 2.1.1. Don de cornée ou de globe oculaire

Dans le cas d'un don de cornée ou de globe oculaire, les capsules cristalliniennes antérieure et postérieure sont découpées sur des cristallins entiers :
- extraits lors d'un prélèvement de cornée ou d'un prélèvement du globe oculaire sur un patient donneur d'organe (le cristallin étant un déchet opératoire lors du geste de prélèvement de cornée ou du globe à but thérapeutique)
- stockés dans un récipient stérile incluant un liquide de conservation - tel qu'un liquide de perfusion oculaire chirurgical ou tout autre milieu stérile de conservation connu de l'homme du métier - pour faciliter leur transport vers un site de mise en œuvre du procédé selon l'invention.

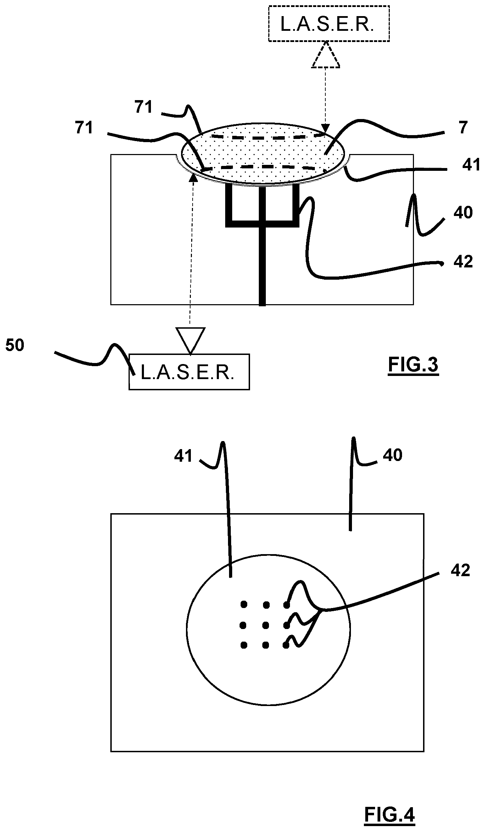

Une fois les cristallins mis à disposition sur le site de mise en œuvre du procédé, les capsules cristalliniennes antérieure et postérieure sont découpées en utilisant par exemple :
- un dispositif de maintien du cristallin permettant l'immobilisation du cristallin 7 lors des opérations de découpe des capsules cristalliniennes antérieure et postérieure ; un tel dispositif de maintien est illustré aux figures 3 et 4 ; il comprend un plot support 40 transparent à un rayonnement L.AS.E.R., le plot support 40 incluant :
   o un logement dont la paroi de réception 41 (i.e. paroi destinée à venir en contact avec le cristallin) est de forme concave complémentaire à la forme du cristallin 7,
   o un (ou plusieurs) conduit(s) d'aspiration 42 dont l'une des extrémités débouche dans la paroi de réception 41, l'autre extrémité du conduit d'aspiration étant destinée à être raccordée à un système d'aspiration (non représenté) de sorte à générer une dépression entre la paroi de réception 41 et le cristallin 7 afin de bloquer le cristallin en position,
- un système de découpe 50 incluant une source L.A.S.E.R. ultrabref (femtoseconde par exemple mais pas uniquement) pour découper les capsules cristalliniennes antérieure et postérieure 71.

En variante, les capsules antérieure et postérieure peuvent être découpées en utilisant un trépan automatisé ou un trépan manuel.

Plus précisément dans le cas d'un don d'organe, le procédé peut comprendre les étapes suivantes préalablement à l'étape de dé-cellularisation 200 :
- insérer le cristallin entier dans un dispositif de maintien du cristallin incluant des première et deuxième faces transparentes au rayonnement L.A.S.E.R.,
- découper un disque de diamètre compris entre 2 et 11 millimètres (ou plusieurs petits disques de petits diamètres) sur la face antérieure de la capsule cristallinienne pour obtenir un (ou plusieurs) disque(s) découpé(s) antérieur(s),

- découper un disque de diamètre compris entre 2 et 11 millimètres (ou plusieurs petits disques de petits diamètres) sur la face postérieure de la capsule cristallinienne pour obtenir un (ou plusieurs) disque(s) découpé(s) postérieur(s),
- disséquer les disques antérieur et postérieur découpés pour obtenir une capsule cristallinienne antérieure et une capsule cristallinienne postérieure,
- stocker de façon stérile chaque capsule cristallinienne ainsi obtenue dans un contenant respectif incluant un liquide de conservation (eau, liquide de perfusion oculaire chirurgical, etc.) préalablement à la mise en œuvre des étapes suivantes du procédé.

Cette source d'approvisionnement régulière permet de découper des capsules cristalliniennes pour obtenir préférentiellement des disques de diamètre supérieur à 6 millimètres. En outre, pour chaque cristallin donné, il est possible de récupérer deux capsules cristalliniennes (capsule antérieure d'une part et capsule postérieure d'autre part), ce qui permet de maximiser la quantité de matière biologique utilisée pour réaliser un matériau de thérapie cellulaire et/ou tissulaire, cette matière biologique étant habituellement inexploitée à des fins thérapeutiques.

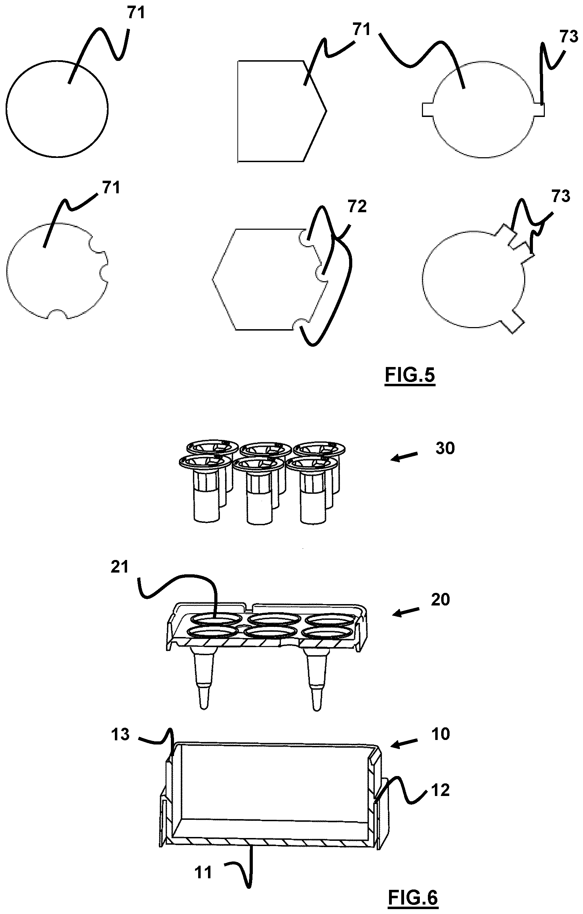

Bien entendu et comme illustré à la figure 5, les capsules cristalliniennes découpées peuvent présenter d'autres formes qu'un disque. Par exemple chaque capsule cristallinienne découpée peut présenter une forme polygonale, pentagonale, carrée, triangulaire, etc. en fonction de l'utilisation visée.

Par ailleurs, chaque capsule découpée peut comprendre des moyens repère d'orientation 72 - tels qu'une (ou plusieurs) encoche(s) - permettant de fournir au praticien une indication sur l'orientation de la capsule 71 (i.e. face avant de la capsule versus face arrière), afin de respecter la courbure naturelle des capsules qui épousent la forme biconvexe du cristallin et de savoir à coup sûr comment orienter le greffon dans l'œil ; notamment dans le cas d'une greffe de capsule re-cellularisée, le repère d'orientation permet au praticien (à la praticienne) de respecter la bonne orientation des cellules qui sont déposées sur la face concave de la capsule (par exemple pour la cornée les cellules endothéliales cornéennes doivent faire face à l'intérieur de l'œil ; pour la rétine, les cellules d'épithélium pigmentées doivent faire face à l'intérieur de l'œil, etc.).

Chaque capsule découpée peut également comprendre des moyens de préhension 73 - tels qu'un (ou plusieurs) ergots de préhension - pour faciliter la manipulation de la capsule. Ces moyens de préhension 73 servent ainsi de « mors » lors de la greffe pour permettre au praticien (à la praticienne) de manipuler sans dommage la capsule lorsqu'elle est recouverte de cellules.

Avantageusement, les moyens repère de position peuvent être intégrés aux moyens de préhension.

### 2.1.2. Rebuts opératoires

Dans le cas de capsules cristalliniennes issues de patients opérés de la cataracte, la capsule cristallinienne antérieure (disque de 5 à 6 millimètres de diamètre le plus souvent) est découpée par le praticien (la praticienne) en conditions stériles à l'aide d'une source L.A.S.E.R. femtoseconde lors de la mise en œuvre de la chirurgie de la cataracte. (ou par un dispositif de découpe électrique ou mécanique dit de capsulorhexis circulaire continu CCC).

Chaque disque de capsule cristallinienne découpé est conditionné dans un contenant stérile incluant un liquide de conservation (tel que de l'eau ou un liquide de perfusion oculaire chirurgical, etc ) afin de les envoyer sur le site de mise en œuvre du procédé selon l'invention.

Pour ces capsules récupérées et donc déjà découpées le procédé peut comprendre, préalablement à l'étape de dé-cellularisation, une étape de découpe supplémentaire (par trépan mécanique ou par L.A.S.E.R.) pour réaliser des marques asymétriques formant des moyens repère d'orientation et/ou des moyens de préhension.

### 2.2. Dé-cellularisation

L'étape de dé-cellularisation 200 permet de supprimer les cellules épithéliales et les fibres cristalliniennes résiduelles fixées à la capsule cristallinienne tout en conservant la structure et la conformation de la capsule cristallinienne. Cette dé-cellularisation permet de diminuer ou de supprimer les risques de réaction immunitaire chez le patient greffé à l'aide du matériau d'allogreffe ou de xénogreffe obtenu en mettant en œuvre le procédé selon l'invention.

L'étape de dé-cellularisation 200 permet d'obtenir une capsule cristallinienne dé-cellularisée :
- qui forme un agent de cicatrisation directement utilisable après réhydratation (pour les défects endothéliaux ou pour les trous maculaires), ou
- qui forme une base matricielle pour une culture cellulaire et/ou tissulaire ultérieure.

L'étape de dé-cellularisation 200 peut mettre en œuvre différentes techniques connues de l'homme du métier basées notamment sur des moyens chimiques (utilisation de fluides appropriés à la dé-cellularisation), et/ou sur des moyens mécaniques (vibrations mécaniques, agitation, grattage, etc.). Les cellules à supprimer étant uniquement à la surface de la capsule, les moyens de dé-cellularisation sont simples (immersion dans l'eau et agitation peuvent suffire par exemple).

Par exemple, dans un mode de réalisation de l'invention, l'étape de dé-cellularisation 200 peut comprendre les sous-étapes suivantes :
- immerger la capsule cristallinienne reçue dans un liquide de dé-cellularisation (incluant par exemple de l'eau pure qui fait éclater les cellules par choc osmotique, du Chlorure de Sodium (NaCl), et/ou de l'Éthylène Diamine Tétra Acétique (EDTA), et/ou un détergent de sodium dodécyl sulfate et/ou une enzyme de type ADNase), et
- optionnellement soumettre la capsule immergée à des vibrations mécaniques et/ou gratter mécaniquement la surface de la capsule cristallinienne, pour retirer les éventuelles cellules épithéliales et/ou fibres cristalliniennes résiduelles fixées à la capsule cristallinienne,
- extraire la capsule cristallinienne et la rincer avec un liquide de rinçage (tel qu'une solution sérum physiologique),
- éventuellement répéter les sous-étapes précédentes pour obtenir une capsule cristallinienne dé-cellularisée.

La mise en œuvre de l'étape de dé-cellularisation 200 permet d'obtenir une capsule cristallinienne dé-cellularisée présentant une bonne biocompatibilité et une bonne résistance biomécanique.

### 2.3. Etape de dépôt sur une membrane support micro poreuse

L'étape de dépôt 300 de la capsule cristallinienne sur une membrane micro poreuse 31 permet d'obtenir un empilement à plat incluant la membrane 31 et la capsule 71. Un tel empilement est facile à manipuler, prêt à l'emploi pour les différentes utilisations (i.e. chirurgie oculaire, ou bioengineering (i.e. ingénierie biologique) cellulaire et tissulaire).

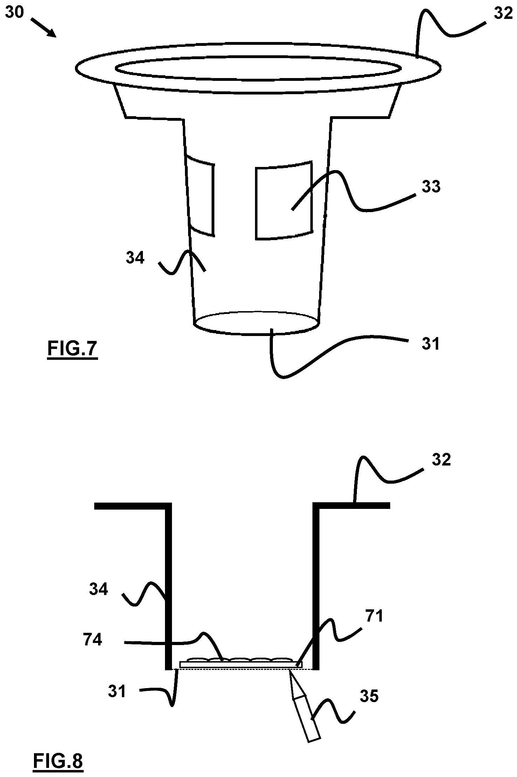

La membrane 31 peut être celle d'un insert de culture cellulaire tel qu'illustré aux figures 6 et 7, ou tout autre dispositif comportant une membrane microporeuse, typiquement une membrane adaptée pour les cultures cellulaires. La membrane microporeuse 31 permet de servir de protection mais aussi permet ultérieurement de cultiver des cellules 74 à la surface de la capsule 71 le cas échéant.

La membrane peut être constituée dans tout matériau connu de l'homme du métier pour réaliser une culture cellulaire/tissulaire. La membrane microporeuse 31 peut être une membrane minérale, en particulier une membrane en Al2O3, TlO2 ou ZrO2 bien que d'autres matériaux minéraux tels que le carbone pyrolytique puissent être utilisés. En variante, la membrane 31 peut être une membrane organique, telle qu'une membrane en polyfluorure de vinylidène, en polycarbonate (PC) ou en polyéthylène téréphtalate (PET).

La membrane 31 est semi transparente, permettant de visualiser facilement la capsule 71 au travers.

Avantageusement, la porosité de la membrane 31 permet de marquer la capsule 71 pour définir un (ou plusieurs) moyens repère d'orientation, avec une encre biocompatible, à travers la membrane 31 et sans avoir à toucher directement la capsule 71. Ceci est utile lors du bioengineering lorsque la capsule 71 est recouverte de cellules 74. On peut alors réaliser un marquage, par exemple avec un feutre chirurgical 35 au violet de crésyl. Ce marquage est de préférence asymétrique tel qu'une lettre asymétrique type F, S ou tel que 3 points périphériques (2 côte à côte, 1 éloigné), sans toucher/léser directement les cellules comme illustré à la figure 8. En variante, le marquage peut être réalisé de façon indélébile dans la matière (par exemple par microperforation mécanique ou au laser ou par gravure au laser femtoseconde etc.).

Avantageusement la porosité de la membrane 31 permet aussi de bien colorer au bleu trypan ou à l'aide de tout autre colorant vital connu de l'homme du métier, la capsules 71 par sa face postérieure ne comportant pas de cellule. Cette coloration facilite la visualisation de la membrane lors des manipulations d'introduction dans l'œil puis de la mise en place à la face postérieure de la cornée ou en face d'un trou maculaire rétinien.

Lors de l'étape de dépôt 300, la capsule cristallinienne est déposée délicatement sur la membrane à laquelle elle adhère spontanément après une simple dessication.

La capsule cristallinienne solidaire de la membrane constitue un empilement qui peut ensuite être lyophilisé, stérilisé, stocké et utilisé pour les différentes applications.

Comme indiqué précédemment, la membrane 31 peut être intégrée à un dispositif de culture cellulaire connu de l'homme du métier, comme par exemple le dispositif de culture cellulaire décrit dans le document WO2018/102329.

En référence aux figures 6 et 7, un tel dispositif de culture cellulaire peut comprendre :
- un réservoir 10 parallélépipédique destiné à contenir un milieu de culture cellulaire ou tissulaire, le réservoir 10 incluant un fond 11 et quatre parois latérales 12 définissant une ouverture supérieure,
- un plateau amovible 20 destiné à être positionné sur le rebord supérieur 13 du réservoir 10, le plateau 20 incluant une pluralité de trous circulaires 21 destinés à recevoir chacun un logement 30 respectif,
- une pluralité de logements 30 incluant chacun une membrane 31, chaque logement 30 étant structuré pour s'ajuster à travers un trou circulaire 21 respectif et se prolonger au-dessous du plateau 20 de sorte que la membrane 31 de chaque logement 30 soit immergée dans le milieu de culture contenu dans le réservoir 10 lorsque le plateau amovible 20 est positionné sur le réservoir 10 et que le logement 30 est inséré dans son trou circulaire 21 associé.

Plus précisément, le logement 30 comprend :
- une collerette supérieure 32 destinée à reposer sur les bords d'un trou 21 respectif du plateau amovible 20,
- une embase fixée à la face inférieure de la collerette 32, l'embase incluant au moins une cloison latérale 33 tronconique allant en se rapprochant dans une direction opposée à la collerette 32, la cloison latérale 33 incluant une (ou plusieurs) fente(s) 34 pour permettre le passage du milieu de culture, et
- la membrane 31 fixée à l'extrémité libre de la cloison latérale 33 et s'étendant dans un plan sensiblement parallèle à un plan contenant la collerette supérieure 32, la membrane 31 incluant une face supérieure et une face inférieure opposée, la face supérieure étant plus proche de la collerette supérieure que la face inférieure.

La membrane 31 peut être amovible. Dans ce cas à l'issue de l'étape de dépôt 300, on obtient un empilement constitué uniquement de la membrane 31 et de la capsule 71.

En variante, la membrane peut être fixée de manière non amovible au logement 30. Dans ce cas à l'issue de l'étape de dépôt 300, on obtient un logement 30 intégrant la membrane 31 et la capsule cristallinienne. Ce logement pourra être ultérieurement utilisé pour réaliser une opération de culture.

### 2.4. Lyophilisation

L'étape de lyophilisation 400 permet de déshydrater (i.e. retirer l'eau contenue dans) les capsules cristalliniennes. Cette étape de lyophilisation permet de faciliter le transport et le stockage ultérieur des matériaux d'allogreffe ou de xénogreffe (obtenus à l'issu du procédé) puisque ceux-ci n'ont plus à être conservés dans un milieu liquide.

L'étape de lyophilisation 400 peut être mise en œuvre à l'aide d'un lyophilisateur (surgélation des capsules cristalliniennes cultivées puis évaporation sous vide de la glace sans la faire fondre, etc.), ou en utilisant toute autre technique de lyophilisation connue de l'homme du métier.

Avantageusement, l'étape de lyophilisation 400 peut être mise en œuvre sur chaque ensemble composé de la membrane 31 et de la capsule cristallinienne (voire sur chaque logement 30 intégrant la membrane 31 et la capsule cristallinienne cultivée). Ceci permet de limiter le nombre d'opérations de manipulation de la capsule cristallinienne, ces opérations de manipulation étant susceptibles d'induire des dégradations de la capsule cristallinienne. En variante, la capsule peut aussi être lyophilisée seule (mais cette lyophilisation est complexe à réaliser car la capsule est fragile).

Une fois la (ou les) capsule(s) cristallinienne(s) lyophilisée(s), celle(s)-ci est (sont) soumise(s) à une étape de stérilisation.

### 2.5. Stérilisation

L'étape de stérilisation 500 permet réduire la quantité de microorganismes susceptibles d'être fixés sur la (ou les) capsule(s) cristallinienne(s) lyophilisée(s). L'étape de stérilisation 500 permet en outre d'augmenter la durée de conservation de la (ou des) capsule(s) cristallinienne(s) lyophilisée(s).

L'étape de stérilisation 500 peut être mise en œuvre par toute technique de stérilisation connue de l'homme du métier telle que l'irradiation (par exemple soumission de chaque capsule cristallinienne lyophilisée à des rayonnements de faisceaux électroniques, des rayonnements gamma ou de la lumière ultraviolette) pendant une certaine période de temps.

Avantageusement, l'étape de stérilisation 500 peut être mise en œuvre sur chaque logement 30 intégrant la membrane 31 et la capsule cristallinienne lyophilisée.

En variante, l'étape de stérilisation 500 peut être mise en œuvre sur l'ensemble composé de la membrane 31 et de la capsule cristallinienne lyophilisée. Dans ce cas, si l'étape de lyophilisation 400 a été effectuée sur le logement 30 intégrant la membrane 31 et la capsule cristallinienne, l'ensemble composé de la membrane 31 et de la capsule cristallinienne lyophilisée peut être retiré du logement 30 préalablement à la mise en œuvre de l'étape de stérilisation 500.

En variante encore, l'étape de stérilisation 500 peut être mise en œuvre uniquement sur la capsule cristallinienne lyophilisée, celle-ci pouvant être aisément décrochée de la membrane 31 après lyophilisation.

### 2.6. Emballage et conditionnement

A l'issue de l'étape de stérilisation 500, soit la capsule cristallinienne stérilisée seule, soit l'empilement composé de la membrane 31 et de la capsule cristallinienne stérilisée, soit le logement 30 intégrant la membrane 31 et la capsule cristallinienne stérilisée est emballé dans un emballage de conditionnement permettant le stockage et le transport des matériaux d'allogreffe ou de xénogreffe obtenus.

### 3. Utilisation des matériaux de greffe

Pour la réalisation d'une greffe, le praticien (la praticienne) reçoit l'emballage contenant les matériaux d'allogreffe ou de xénogreffe.

Une fois le matériau sorti de son emballage, celui-ci doit être réhydraté.

Pour ce faire, le praticien (la praticienne) peut réhydrater la capsule cristallinienne lyophilisée et stérilisée :
- soit directement dans son logement (si celui-ci a été emballé avec la capsule cristallinienne),
- soit sur sa membrane (si seul l'ensemble composé de la membrane et de la capsule a été emballé),
- soit réhydrater uniquement la capsule cristallinienne (si celle-ci a été emballée seule ou a été décrochée de la membrane préalablement à l'opération de réhydratation).

La réhydratation 610 de la capsule cristallinienne peut être réalisée en utilisant de l'eau, ou du sérum physiologique ou un liquide de perfusion intraoculaire de chirurgie ophtalmologique ou tout autre liquide connu de l'homme du métier.

Une fois le matériau d'allogreffe ou de xénogreffe réhydraté, la procédure de greffe peut être mise en œuvre de manière classique.

### 3.1. Greffe d'une capsule dé-cellularisée

Pour certaines applications, la capsule cristallinienne sortie de son emballage peut être greffée 620 directement après réhydratation 610.

Par exemple, la capsule dé-cellularisée et réhydratée peut être greffée derrière une cornée atteinte de dystrophie de Fuchs et dont le chirurgien a, au préalable, ôté la membrane de Descemet. La capsule ainsi greffée permet de guider la repousse in *vivo* des cellules endothéliales.

En variante, la capsule dé-cellularisée et réhydratée peut être greffée pour boucher un trou maculaire rétinien. Dans ce cas, la capsule greffée permet de favoriser la cicatrisation (la capsule joue le même rôle de cicatrisant que les lambeaux de membrane limitante interne que le chirurgien peut mettre dans le trou maculaire pour favoriser sa cicatrisation).

Bien entendu, la greffe de capsule cristallinienne dé-cellularisée et réhydratée peut être mise en œuvre pour tout autre application connue de l'homme du métier.

### 3.2. Greffe d'une capsule recouverte de cellules

Pour d'autres applications, la capsule cristallinienne réhydratée peut être greffée 640 postérieurement à la mise en œuvre d'une étape de culture 630 : culture cellulaire in *vitro* à la surface de chaque capsule cristallinienne dé-cellularisée/lyophilisée/stérilisée/réhydratée pour obtenir des capsules cristalliniennes cultivées ; à titre d'exemples non limitatif :
o cellules endothéliales cornéennes pour des reconstructions de l'endothélium cornéen (appelés TEEK pour Tissu Engineered Endothelial Keratoplasty),
o cellules épithéliales cornéennes pour des reconstructions de surface oculaire,
o cellules de l'épithélium pigmenté de la rétine pour des maladies comme certaines rétinites pigmentaires atteignant spécifiquement cette couche ou pour des maladies fréquentes comme la dégénérescence maculaire liée à l'âge (DMLA),
o photorécepteur (cône et/ou bâtonnet) pour des rétinites pigmentaires,
o cellules ganglionnaires pour des neuropathies optiques.

L'étape de mise en culture par les cellules cibles (cornéennes, rétiniennes, etc) 630 des capsules cristalliniennes réhydratées (dé-cellularisées de leurs propres cellules) permet d'obtenir des capsules cristalliniennes cultivées susceptibles d'être greffées sur un patient (bioengineering).

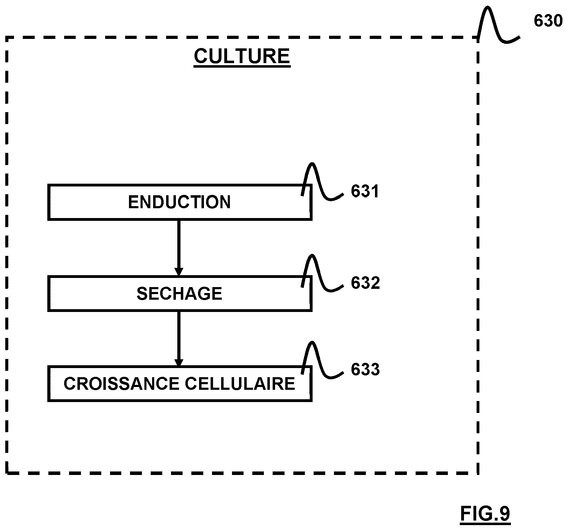

Comme illustré à la figure 9, l'étape de culture 630 peut comprendre une sous-étape d'enduction 631 (ou *« coating »* en anglais) de la capsule cristallinienne avec une substance favorisant l'adhérence des cellules/tissus cibles destinés à être cultivés sur la capsule cristallinienne dé-cellularisée - telle qu'une substance composée de fibronectine, laminine, protéoglycannes) ou tout autre substance connue de l'homme du métier.

Cette sous-étape d'enduction 631 peut être suivie (si besoin) d'une sous-étape de séchage 632 préalablement à la culture cellulaire ou tissulaire.

Enfin, l'étape de culture 630 comprend une sous-étape consistant à faire croître 633 des cellules/tissus sur la capsule cristallinienne dé-cellularisée :
- le réservoir 10 du dispositif de culture est rempli de liquide de culture,
- le plateau amovible 20 est installé sur le réservoir 10, et
- chaque logement 30 est installé à l'intérieur d'un trou circulaire 21 respectif.

Une fois la culture réalisée, le matériau obtenu peut être utilisé pour réaliser une greffe.

### 4. Essais

L'essai suivant a été effectué par les inventeurs pour démontrer la possibilité de culture cellulaire sur une capsule cristallinienne dé-cellularisée.

### 4.1. Objectif

L'objectif est d'ensemencer des Cellules Endothéliales Cornéennes (ci-après dénommées « *CEC* ») sur une capsule cristallinienne afin d'évaluer l'intérêt de son utilisation en tant que support biologique pour la bio-ingénierie d'un greffon endothélial.

### 4.2. Matériel

Des capsules cristalliniennes de 6 millimètres de diamètre (rebuts opératoires issus de patients opérés de la cataracte avec découpe de la capsule au laser femtoseconde) ont été utilisées. Ces capsules cristalliniennes ont été dé-cellularisées, stérilisées et conservées dans de l'eau pure à 4°C avant leur utilisation.

Des CEC en culture primaire au 5^{ème} passage ont été utilisées.

### 4.3. Endothélialisation

Les capsules cristalliniennes de 6 millimètres de diamètre ont été déposées sur la membrane d'insert de culture cellulaire de type Nunc, de boite 24 puits. La membrane en polycarbonate était micro-perforée et avait une porosité de 0,4mm. L'adhérence de la capsule à la membrane a été obtenu par séchage simple. Les capsules sur les membranes des inserts ont été lyophilisées, puis stockées à température ambiante dans une boite hermétique.

Le jour de l'utilisation pour la cellularisation, la capsule a été réhydratée par immersion de l'insert de culture dans un puits contenant du Phosphate Buffer Saline.

Un milieu d'enduction (« *coating »)* a été ajouté (laminine LMN511). Les cellules ont été ensemencées à 5000 cellules/mm².

Sept jours après le début de mise en culture in vitro des capsules cristalliniennes dans la solution de CEC, les membranes des CEC ont été visualisées au microscope optique a transmission après coloration au rouge alizarine et au microscope confocal après coloration au Dioc6 (3,3'-dihexyloxacarbocyanine iodide, colorant fluorescent de membranes des organites intracellulaire) qui permet la visualisation spécifique du cytoplasme et au Dapi (4',6-diamidino-2-phenylindole), spécifique aux noyaux.

### 4.4. Résultats

La coloration au rouge alizarine a montré une couverture totale de la surface de la capsule cristallinienne par les CEC avec une Densité Cellulaire Endothéliale (DCE) résiduelle d'environ 3000 cellules/mm².

L'observation au microscope confocal a confirmé la formation d'une monocouche cellulaire tapissant uniformément la surface de la capsule cristallinienne

La capsule cristallinienne a permis la formation d'une monocouche cellulaire de CEC à sa surface tout en gardant une excellente transparence et solidité.

Des greffes endothéliales expérimentales ont été réalisées sur le lapin en utilisant un TEEK composé d'une capsule cristallinienne humaine traitée comme décrit ci-dessus et endothélialisée avec des CEC humaines.

Le jour de la greffe, la partie amovible de l'insert de culture cellulaire comportant un fin anneau de plastique et la membrane polycarbonate sur laquelle adhère la capsule cristallinienne endothélialisée, a été saisie stérilement à la main. Le TEEK est ainsi manipulable sans aucun traumatisme. La face de la membrane polycarbonate a été séchée à l'aide d'une microéponge chirurgicale. Trois marquages asymétriques ont été réalisées sur le bord du TEEK (bien visible par transparence à œil nu) et par sa face arrière au travers de la membrane polycarbonate afin de repérer le sens (face endothélialisée versus face sans cellules) lors de la greffe dans l'œil receveur.

Le TEEK a ensuite été coloré au bleu trypan 0,4% (en suivant le même protocole que lors d'une greffe endothéliale habituelle) directement dans l'insert de culture. Le colorant a été appliqué des deux côtés de la membrane pour bien imprégner le greffon par les 2 faces.

La membrane en polycarbonate a été détachée de son anneau de plastique à l'aide d'une lame de bistouri ou d'un trépan de diamètre supérieur au TEEK. Le TEEK peut ainsi être manipulé sans aucun contact puisque seule la membrane est tenue avec des pinces du chirurgien.

L'ensemble membrane + TEEK a été immergé dans un liquide d'irrigation chirurgical usuel de type Balanced Salt Solution (BSS). Le TEEK a été détaché de la membrane à l'aide d'une spatule permettant de soulever le bord du TEEK. La coloration bleue du TEEK aide à bien le visualiser durant cette phase effectuée sous microscope opératoire (Le TEEK peut à nouveau être recoloré si besoin au bleu trypan si le chirurgien le juge nécessaire).

Le TEEK s'est enroulé sur lui-même pour former un rouleau avec les CEC à l'extérieur, à la façon d'un greffon endothélial naturel de donneur de type DMEK (Descemet Membrane Endothelial Keratoplasty).

Le TEEK é été manipulé comme un greffon de DMEK, introduit dans un injecteur en verre ou une cartouche d'injection en plastique et injecté dans l'œil receveur selon la procédure chirurgicale usuelle.

Il s'est déroulé dans l'œil par des manœuvres chirurgicales usuelles. La bonne orientation (face endothélialisée dirigée vers l'intérieure de l'œil et face sans cellules contre la cornée) a été vérifiée grâce aux marques colorées asymétriques et/ou aux trois encoches et/ou trois mors présents à la périphérie du TEEK.

Le TEEK a été maintenu en place par une bulle de gaz (air ou mélange 80% d'air et 20% de gaz SF6 (hexafluorure de soufre)) comme lors d'une greffe endothéliale chez l'homme.

Le bon fonctionnement du TEEK a ensuite été confirmé par sa capacité à maintenir fine et transparente pendant 4 semaines la cornée du lapin au préalablement rendu pathologique par destruction de ses propres CEC.

Des lapins témoins greffés avec une capsule mais SANS cellularisation par les CEC ont servi de contrôle, leurs cornées ne se sont pas améliorées et sont restés œdémateuses.

### 5. Conclusions

Le procédé de préparation décrit précédemment permet d'obtenir un matériau d'allogreffe ou de xénogreffe à partir d'une capsule cristallinienne.

La capsule cristallinienne dé-cellularisée est un tissu non immunogène, et par définition biocompatible. Elle est aussi transparente et constitue un excellent support de culture cellulaire.

L'origine humaine est garante d'une acceptation facilitée par les autorités de santé. Il est possible de sécuriser le don par un diagnostic sérologique des donneurs (un tel diagnostic est déjà obligatoire pour les dons de cornée, il sera facile à obtenir pour les opérés de la cataracte).

La principale utilisation des greffons obtenus en mettant en œuvre le procédé selon l'invention concerne le traitement de pathologies oculaires. Ces greffons peuvent être :
- des greffons endothéliaux ; les greffons obtenus à l'aide du procédé selon l'invention reproduisent fidèlement les greffons préparés à partir de cornées de donneurs type DMEK (Descemet Membrane Endothelial Keratoplasty) ou TEEK (Tissue Engineered Endothelial Keratoplasty) : ils ne changent pas les habitudes chirurgicales et leur nombre n'est pas limité,
- d'autres greffons (épithéliaux, conjonctivaux, rétiniens) pour apporter des cellules souches ou différentiées sur un support transparent, facile à manipuler
- des greffons de guidage (« pansement descemétique » pour traiter les dysfonctions endothéliales en guidant la cicatrisation) ou de cicatrisation (cicatrisation de Trous maculaires).

Le lecteur appréciera que le dépôt des capsules cristalliniennes reçues sur une membrane d'un logement respectif de façon individuelle permet de faciliter toute la chaine de traitement de la capsule cristallinienne depuis sa dé-cellularisation jusqu'à sa réhydratation en vue d'une greffe. En effet, l'utilisation d'un logement intégrant une membrane support sur laquelle la capsule cristallinienne est déposée permet de limiter les risques de détérioration de la capsule cristallinienne (en limitant les contacts directs de la capsule cristallinienne par les différents opérateurs).

Le lecteur aura compris que de nombreuses modifications peuvent être apportées à l'invention décrite précédemment sans sortir matériellement des nouveaux enseignements et des avantages décrits ici.

Par exemple, le procédé peut comprendre une étape de marquage des capsules pour indiquer une orientation, soit par découpe mécanique (trépan ou L.A.S.E.R.) soit par marquage à l'encre biocompatible avec un signe asymétrique, permettant de différentier les deux faces de la capsule cristallinienne (notamment lorsque l'une des faces porte des cellules, cette dernière étant celle devant être mise au contact de la cornée malade receveuse).

## Revendications

1. Procédé de préparation d'un matériau d'allogreffe ou de xénogreffe à partir d'une capsule cristallinienne, ***caractérisé en ce que*** le procédé comprend les étapes suivantes :
- Dé-cellularisation (200) d'une capsule cristallinienne (71) pour obtenir une capsule cristallinienne dé-cellularisée,
- Dépôt (300) de la capsule dé-cellularisée sur une membrane support (31) microporeuse pour obtenir un empilement composé de la capsule cristallinienne dé-cellularisée et de la membrane support,
- Marquage de la capsule cristallinienne dé-cellularisée pour former des moyens repère d'orientation, ladite étape de marquage consistant en l'application d'une encre biocompatible à travers la membrane support (31),
- Lyophilisation (400) de l'empilement pour obtenir un empilement lyophilisé,
- Stérilisation (500) de l'empilement lyophilisée pour obtenir un empilement stérilisé,
- Emballage (600) de l'empilement stérilisé pour obtenir des matériaux d'allogreffe ou de xénogreffe.

2. Procédé de préparation selon la revendication 1, lequel comprend en outre une étape de découpe de la capsule cristallinienne (71) pour former des moyens de préhension (73).

3. Procédé de préparation selon l'une quelconque des revendications 1 ou 2, lequel comprend, préalablement à l'étape de dé-cellularisation :
- une étape de réception d'un matériel biologique primaire choisi parmi : un globe oculaire ou une cornée issu d'un don d'organe, ou une capsule cristallinienne issue d'une opération de la cataracte et constituant un rebut opératoire,
- si le matériau biologique est un globe ou une cornée :
o une étape d'extraction du cristallin (7),
o une étape de découpe des capsules cristalliniennes (71) antérieure et postérieure.

4. Procédé de préparation selon la revendication 3, dans lequel l'étape de découpe des capsules cristalliniennes antérieure et postérieure comprend les sous-étapes consistant à :
- insérer le cristallin entier dans un dispositif de maintien incluant un logement transparent au rayonnement L.A.S.E.R., et des moyens d'aspiration pour plaquer le cristallin dans le logement,
- découper en utilisant un rayonnement L.A.S.E.R., la face antérieure de la capsule cristallinienne pour obtenir un disque découpé antérieur,
- découper en utilisant le rayonnement L.A.S.E.R., la face postérieure de la capsule cristallinienne pour obtenir un disque découpé postérieur,
- disséquer les disques antérieur et postérieur découpés pour obtenir une capsule cristallinienne antérieure et une capsule cristallinienne postérieure.

5. Procédé de préparation selon l'une quelconque des revendications 1 à 4, ***dans lequel*** l'étape de dé-cellularisation (200) comprend les sous-étapes suivantes consistant à :
- immerger la capsule cristallinienne dans un liquide de dé-cellularisation, et
- optionnellement soumettre la capsule immergée à des vibrations mécaniques et/ou gratter mécaniquement la surface de la capsule cristallinienne,
- rincer la capsule cristallinienne avec un liquide de rinçage (tel qu'une solution sérum physiologique).

6. Procédé de préparation selon l'une quelconque des revendications 1 à 5, dans lequel l'étape de dépôt (300) comprend les sous-étapes consistant à :
- positionner la capsule cristallinienne à plat sur la membrane support,
- effectuer une dessication de la capsule cristallinienne pour favoriser l'adhérence de la capsule cristallinienne sur la membrane support.

7. Procédé de préparation selon l'une quelconque des revendications 1 à 6, ***lequel*** comprend en outre, postérieurement à l'étape d'emballage, une étape de culture cellulaire ou tissulaire, ladite étape de culture incluant les sous-étapes consistant à :
- enduire la capsule cristallinienne avec une substance favorisant l'adhérence des cellules/tissus destinés à être cultivés,
- faire croître (340) des cellules/tissus sur la capsule cristallinienne.

8. Procédé de préparation selon l'une quelconque des revendications 1 à 7, ***lequel*** comprend en outre, postérieurement à l'étape d'emballage, une étape de réhydratation du matériau d'allogreffe ou de xénogreffe, pour permettre l'utilisation de la capsule cristallinienne soit directement en tant qu'agent cicatrisant, soit postérieurement à la mise en œuvre d'une étape de culture cellulaire ou tissulaire.

9. Kit chirurgical pour le traitement d'une pathologie oculaire, ***caractérisé en ce que*** le kit comprend un matériau d'allogreffe ou de xénogreffe obtenu en mettant en œuvre le procédé selon l'une des revendications 1 à 8.

## Patentansprüche

1. Verfahren zur Herstellung eines Allotransplantats oder Xenotransplantats aus einer Linsenkapsel, **dadurch gekennzeichnet, dass** das Verfahren die folgenden Schritte umfasst:
- Dezellularisieren (200) einer Linsenkapsel (71), um eine dezellularisierte Linsenkapsel zu erhalten,
- Ablegen (300) der dezellularisierten Kapsel auf einer mikroporösen Trägermembran (31), um einen Stapel zu erhalten, der aus der dezellularisierten Linsenkapsel und der Trägermembran zusammengesetzt ist,
- Markieren der dezellularisierten Linsenkapsel, um Orientierungsmittel zu bilden, wobei der Markierungsschritt aus dem Auftragen einer biokompatiblen Tinte durch die Trägermembran (31) besteht,
- Lyophilisieren (400) des Stapels, um einen lyophilisierten Stapel zu erhalten,
- Sterilisieren (500) des lyophilisieren Stapels, um einen sterilisierten Stapel zu erhalten,
- Verpacken (600) des sterilisierten Stapels, um ein Allotransplantat oder ein Xenotransplantat zu erhalten.

2. Herstellungsverfahren nach Anspruch 1, das ferner einen Schritt des Schneidens der Linsenkapsel (71) umfasst, um Greifmittel (73) zu bilden.

3. Herstellungsverfahren nach einem der Ansprüche 1 oder 2, das vor dem Dezellularisierungsschritt umfasst:
- einen Empfangsschritt eines primären biologischen Materials, das ausgewählt ist aus: einem Augapfel oder einer Hornhaut aus einer Organspende, oder einer Linsenkapsel aus einer Kataraktoperation, die einen Operationsabfall darstellt,
- wenn das biologische Material ein Augapfel oder eine Hornhaut ist:
o einen Extraktionsschritt der Linse (7),
o einen Schneideschritt der vorderen und hinteren Linsenkapsel (71).

4. Herstellungsverfahren nach Anspruch 3, wobei der Schritt des Schneidens der vorderen und hinteren Linsenkapsel die Unterschritte umfasst, die darin bestehen:
- Einsetzen der gesamten Linse in eine Haltevorrichtung, die eine für L.A.S.E.R.-Strahlung durchlässige Aufnahme und Saugmittel zum Anlegen der Linse in der Aufnahme einschließt,
- Schneiden der Vorderseite der Linsenkapsel unter Verwendung einer L.A.S.E.R.-Strahlung, um eine vordere geschnittene Scheibe zu erhalten,
- Schneiden der Rückseite der Linsenkapsel unter Verwendung von L.A.S.E.R.-Strahlung, um eine hintere geschnittene Scheibe zu erhalten,
- Sezieren der vorderen und hinteren Scheibe, um eine vordere Linsenkapsel und eine hintere Linsenkapsel zu erhalten.

5. Herstellungsverfahren nach einem der Ansprüche 1 bis 4, wobei der Dezellularisierungsschritt (200) die folgenden Unterschritte umfasst, die darin bestehen:
- Eintauchen der Linsenkapsel in eine Dezellularisierungsflüssigkeit, und
- optional Aussetzen der eingetauchten Kapsel mechanischen Vibrationen und/oder mechanisches Abkratzen der Oberfläche der Linsenkapsel,
- Spülen der Linsenkapsel mit einer Spülflüssigkeit (wie beispielsweise einer Kochsalzlösung).

6. Herstellungsverfahren nach einem der Ansprüche 1 bis 5, wobei der Schritte des Ablegens (300) die Unterschritte umfasst, die darin bestehen:
- Positionieren der Linsenkapsel flach auf der Trägermembran,
- Durchführen einer Trocknung der Linsenkapsel, um die Haftung der Linsenkapsel an der Trägermembran zu fördern.

7. Herstellungsverfahren nach einem der Ansprüche 1 bis 6, das ferner nach dem Verpackungsschritt einen Zell- oder Gewebskulturschritt umfasst, wobei der Kulturschritt die Unterschritte einschließt, die darin bestehen:
- Beschichten der Linsenkapsel mit einer Substanz, die die Haftung von Zellen/Geweben, die kultiviert werden sollen, fördert,
- Wachsenlassen (340) von Zellen/Gewebe auf der Linsenkapsel.

8. Herstellungsverfahren nach einem der Ansprüche 1 bis 7, das ferner nach dem Verpackungsschritt einen Rehydrierungsschrit des Allotransplantats oder des Xenotransplantats umfasst, um die Verwendung der Linsenkapsel entweder direkt als Wundheilmittel oder nach der Durchführung eines Schritts der Zell- oder Gewebekultur zu ermöglichen.

9. Chirurgisches Kit zur Behandlung einer Augenerkrankung, **dadurch gekennzeichnet, dass** das Kit ein Allotransplantat oder Xenotransplantat umfasst, das durch die Durchführung des Verfahrens nach einem der Ansprüche 1 bis 8 erhalten wird.

## Claims

1. A preparation process for preparing an allograft or xenograft material from a lens capsule, ***characterized in that*** the process comprises the following steps:
- Decellularizing (200) a lens capsule (71) to obtain a decellularized lens capsule,
- Depositing (300) the decellularized capsule on a microporous support membrane (31) to obtain a stack composed of the decellularized lens capsule and the support membrane,
- Marking the decellularized lens capsule to form orientation marker means, said marking step consisting of applying a biocompatible ink through the support membrane (31),
- Lyophilizing (400) the stack to obtain a lyophilized stack,
- Sterilizing (500) the lyophilized stack to obtain a sterilized stack,
- Packaging (600) the sterilized stack to obtain allograft or xenograft materials.

2. The preparation process as claimed in claim 1, which further comprises a step of cutting the lens capsule (71) to form gripping means (73).

3. The preparation process as claimed in any one of claims 1 or 2, which comprises, prior to the decellularization step:
- a step of receiving a primary biological material selected from: a globe or a cornea from an organ donation, or a lens capsule from a cataract operation and constituting surgical waste,
- if the biological material is a globe or cornea:
o a step of extracting the lens (7),
o a step of cutting the anterior and posterior lens capsules (71).

4. The preparation process as claimed in claim 3, wherein the step of cutting the anterior and posterior lens capsules comprises the substeps consisting in:
- inserting the entire lens into a holding device including a housing transparent to laser radiation, and suction means for pressing the lens into the housing,
- cutting the anterior face of the lens capsule using laser radiation to obtain a cut anterior disk,
- cutting the posterior face of the lens capsule using laser radiation to obtain a cut posterior disk,
- dissecting the cut anterior and posterior disks to obtain an anterior and a posterior lens capsule.

5. The preparation process as claimed in any one of claims 1 to 4, ***wherein*** the decellularization step (200) comprises the following substeps consisting in:
- immersing the lens capsule in a decellularizing liquid, and
- optionally subjecting the immersed capsule to mechanical vibrations and/or mechanically scraping the surface of the lens capsule,
- rinsing the lens capsule with a rinsing liquid (such as saline solution).

6. The preparation process as claimed in any one of claims 1 to 5, wherein the deposition step (300) comprises the substeps consisting in:
- positioning the lens capsule flat on the support membrane,
- desiccating the lens capsule to promote adhesion of the lens capsule to the support membrane.

7. The preparation process as claimed in any one of claims 1 to 6, ***which*** further comprises, after the packaging step, a cell or tissue culture step, said culture step including the substeps consisting in:
- coating the lens capsule with a substance that promotes the adhesion of the cells/tissues to be cultured,
- growing (340) cells/tissues on the lens capsule.

8. The preparation process as claimed in any one of claims 1 to 7, ***which*** further comprises, after the packaging step, a step of rehydrating the allograft or xenograft material, to allow the use of the lens capsule either directly as a healing agent, or after the implementation of a cell or tissue culture step.

9. A surgical kit for treating ocular pathology, ***characterized in that*** the kit comprises an allograft or xenograft material obtained by implementing the process as claimed in one of claims 1 to 8.
